# EUROPEAN PATENT SPECIFICATION

(11) **EP 0 741 135 B1**
(45) Date of publication and mention of the grant of the patent: **21.07.1999**
(21) Application number: 96111772.8
(22) Date of filing: 10.04.1992
(51) Int. Cl.: C07F 9/38, C07F 9/6571

(54) **6H-Dibenz(c,e)(1,2)oxaphosphorin-6-oxide derivatives for the stabilisation of 3,4-epoxycyclohexylmethyl (meth)acrylate**
6H-Dibenz(c,e)(1,2)oxaphosphorin-6-oxid Derivative zur Stabilisierung von 3,4-Epoxycyclohexylmethyl(meth)acrylat
Dérivés de 6H-dibenz(c,e)(1,2)oxaphosphorin-6-oxide pour la stabilisation de 3,4-époxycyclohexylméthyl (méth)acrylate

(30) Priority: 28.08.1991 JP 21679891; 05.12.1991 JP 32172491
(43) Date of publication of application: 06.11.1996
(62) Divisional of application: 92106244.4
(73) Proprietor: DAICEL CHEMICAL INDUSTRIES, LTD., Osaka-fu 590 (JP)
(72) Inventor: Kuwana, Akihiro, Otake-shi, Hiroshima-ken (JP); Honda, Kimihide, Himeji-shi, Hyogo-ken (JP); Koga, Kunio, Himeji-shi, Hyogo-ken (JP)
(74) Representative: Hansen, Bernd, Dr. Dipl.-Chem.

(56) References cited:
- PATENT ABSTRACTS OF JAPAN vol. 009, no. 278 (C-312), 6 November 1985 & JP-A-60 123547 (KYOWA GAS KAGAKU KOGYO KK), 2 July 1985,
- PATENT ABSTRACTS OF JAPAN vol. 016, no. 560 (C-1008), 2 December 1992 & JP-A-04 217674 (DAICEL CHEM IND.), 7 August 1992,
- CHEMICAL ABSTRACTS, vol. 96, no. 19, 10 May 1982 Columbus, Ohio, US; abstract no. 162451, XP002012306 & ZH. ORG. KHIM., vol. 18, no. 1, 1982, pages 90-94,
- PATENT ABSTRACTS OF JAPAN vol. 013, no. 105 (C-575), 13 March 1989 & JP-A-63 280728 (NIPPON ESTER CO LTD), 17 November 1988,
- PATENT ABSTRACTS OF JAPAN vol. 009, no. 091 (C-277), 19 April 1985 & JP-A-59 222496 (SANKOU KAIHATSU KAGAKU KENKYUSHO:KK), 14 December 1984,
- PATENT ABSTRACTS OF JAPAN vol. 012, no. 410 (C-540), 28 October 1988 & JP-A-63 150352 (UNITIKA LTD), 23 June 1988,
- PATENT ABSTRACTS OF JAPAN vol. 015, no. 508 (C-0897), 24 December 1991 & JP-A-03 223354 (CHISSO CORP), 2 October 1991,
- CHEMICAL ABSTRACTS, vol. 114, no. 18, 6 May 1991 Columbus, Ohio, US; abstract no. 165087, XP002012307 & JP-A-02 262 574 (DAICEL CHEMICAL INDUSTRY) 25 October 1990
- English translation of JP-A-2191267 .

## Description

### FIELD OF THE INVENTION

The present invention relates to a composition comprising 3,4-epoxycyclohexyl methyl (meth)acrylate.

### BACKGROUND OF THE INVENTION

Heretofore, various acrylate monomers such as methylacrylate, ethylacrylate, 2-ethylhexyl acrylate, etc., which are monofunctional monomers, and trimethylolpropane triacrylate, pentaerythritol triacrylate, etc., which are multifunctional monomers have been widely known.

However, the monofunctional monomers have a disadvantage in that an odor of the residual monomer remaining after curing causes a remarkable problem when the monomers are used as a component of printing inks or coatings.

Furthermore, the multifunctional monomers also have a disadvantage that it is necessary to use large amounts of monomers to resins when using the monomer as a diluent of printing inks or coatings, resulting in loss of excellent properties of the resins.

On the other hand, cyclohexyl methyl (meth)acrylate itself is readily polymerized or copolymerized with other compounds having unsaturated groups by heat, ultraviolet rays and or ionized radiation in the presence of an initiator for free radical polymerization.

In particular, an epoxidized cyclohexyl methyl (meth)acrylate which has an alicyclic epoxy group capable of being cured by a cation, that is, 3,4-epoxycyclohexyl methyl (meth)acrylate is useful for polymerizing or copolymerizing.

3,4-epoxycyclohexyl methyl (meth)acrylate is low in viscosity and mild in odor, and has a wide range of solubility to resins, and further it is useful for inks, coatings, adhesives, covering agents and as a raw material for molding resins or a modifier thereof.

It is noted that 3,4-epoxycyclohexyl methyl (meth)acrylate and a process for the preparation thereof is generally known. Specifically the esterification reaction of tetrahydrobenzyl alcohol with (meth)acrylic acid or the transesterification reaction of tetrahydrobenzyl alcohol with a (meth)acrylate ester and successively by the epoxidation reaction with a peracid [Batog, A. E.; Zaitsev, S. Yu.; Kiryushima, N. P.; Zaitseva, V. V. (Inst.Fiz.-Org. Khim. Uglechim., Donetsk, USSR). Zh. Org. Khim, 1982, 18(1), 90-4 (Russ)] are known.

The reaction schemes are represented by the following formulae; 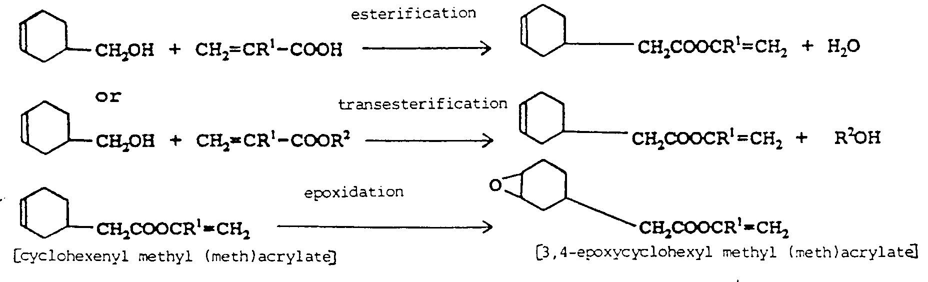 [in the formulae, R¹ is hydrogen or a methyl group, R² is an alkyl group].

However, a process for the preparation of a purified, that is, a commercially useful 3,4-epoxycyclohexyl methyl (meth)acrylate, has not been disclosed up to date.

In particular, there has not been known a process in which even a waste water treatment is taken into consideration or even a small amount of impurity detected by a heptane test described hereinafter can be removed.

On the other hand, it has been known that 3,4-epoxycyclohexyl methyl (meth)acrylate has a disadvantage of exceedingly readily polymerizing, particularly, during the preparation processes, while being stored and/or shipped under the influence of heat, light or other causes.

In order to solve this disadvantage, Japanese Patent Unexamined Publication (Kokai) No. 262,574/1990 teaches a method for preventing polymerization, which uses quinones, etc. together with phosphorous compounds in the presence of molecular state oxygen gas.

JP-A-60123547 discloses a methacrylic copolymer composition in which a phosphaphenanthrene-based compound is mixed with a methacrylic copolymer in order to thermally stabilize the methacrylic copolymer. It is employed for preventing discoloration in a (meth)acrylic resin. Particularly, it improves the thermal stability in moulded articles prepared from a (meth)acrylic resin.

Further, JP-A-59222496 discloses a process for the preparation of the above compound, and it teaches that the compound is effective for the prevention of deterioration and discoloration in a variety of organic chemicals. However, it is not disclosed that the compound is also effective for preventing an increase of heptane test (HT) value in an epoxy (meth)acrylate monomer.

However, it has been found by the present inventors that the use of polymerization inhibitors as described hereinabove is not sufficient in the case of commercial preparation processes.

One of the reasons why the effect of this method was not sufficient in that there was not sufficient qualities of 3,4-epoxycyclohexyl methyl (meth)acrylate possessed, since it was not produced on a commercial basis when the Japanese Publication was filed.

Specifically, it is required that low boiling components in a commercially available 3,4-epoxycyclohexyl methyl (meth)acrylate must be removed to the extent of from 2 to 3 %, more preferably, not more than 1 %.

For that purpose, it is required that heating temperatures be raised and/or that processing time be extended during the step of removing the low-boiling ingredients.

However, raising the temperatures or extension of processing time generates, even though in minor amounts, polymers in the product.

It has now been found that the polymers in the product cause problems even though present in minor amounts in commercially available 3,4-epoxycyclohexyl methyl (meth)acrylate.

For example, one of the problems is that the polymers ooze out as adhesive and insoluble substances when preparing intermediate materials of resins for coatings using 3,4-epoxycyclohexyl methyl (meth)acrylate including the polymers. This results in causing various problems in processing and in coatings having a remarkably lower commercial valuation being produced.

It appears that the minor amounts of polymers in the commercially available 3,4-epoxycyclohexyl methyl (meth)acrylate are composed of low molecular weight polymers of 3,4-epoxycyclohexyl methyl (meth)acrylate itself.

The amount of such low molecular weight polymers can be determined in weight % with a measuring method using n-heptane or n-hexane. 10 g of a product is dissolved in 100 cc of n-heptane or n-hexane and the resulting suspension is filtered and weighed (hereinafter, occasionally referred to as HT or HT value in a solubility test).

It is known that a commercially available 3,4-epoxycyclohexyl methyl (meth)acrylate must exhibit an HT value of not more than 0.1 % by weight.

It was found that the method described in Japanese Patent Unexamined Publication (Kokai) No. 262594 / 1990 can only provide 3,4-epoxycyclohexyl methyl (meth)acrylate having a polymer content of more than 0.1 % by weight in HT value, more specifically, 0.14 % by weight or so. These values are not sufficient by a recollected confirmation test using HT carried out thereafter.

That is, further more effective methods for inhibiting polymerizing in each step of the preparation process must be developed in order to produce a purified 3,4-epoxycyclohexyl methyl (meth)acrylate on a commercial basis.

It is noted that 3,4-epoxycyclohexyl methyl (meth)acrylate has an alicyclic epoxy group which tends to exceedingly readily react with an organic acid derived from an organic peracid epoxidation agent. For example, the epoxy group reacts with acetic acid derived from peracetic acid in the case of using peracetic acid as an organic peracid, resulting in polymerization of 3,4-epoxycyclohexyl methyl (meth)acrylate and opening of the epoxy group, particularly in the evaporation step.

Accordingly, it is necessary to remove the organic acid from the crude reaction solution as early as possible in order to maintain a short period of time of contact of the acid with the epoxy group.

More effective methods have not been developed to date.

Furthermore, it is noted that, heretofore, various processes for removing the organic acid and organic peracid have been tried.

In the case of a heat resistant product, a refining process by distillation has usually been carried out (process (a)).

The organic acid or organic peracid which are dissolved in the crude reaction solution can be removed by extraction with water and successively by distillation, in order to prevent the polymerization or the side reaction of an epoxy compound on distilling the crude reaction solution without any refining processes (process (b)).

In cases where the organic acid or organic peracid cannot be removed or where the organic acid in an aqueous solution readily reacts with the epoxy compound, a neutralization process has been usually applied (process (c)).

Furthermore, where the substances which cause the polymerization and the side reaction cannot be removed by merely adjusting the PH of the solution to neutral, the substances are occasionally removed with an aqueous alkali solution.

Distillation is carried out in order to refine after removing the substances by neutralization.

However, the prior art process (a) is often incapable of being applied, because it has a disadvantage in that distillation alone can cause the polymerization or the opening reaction of epoxy groups because of ease of the reaction of epoxy groups with organic acid.

Furthermore, the prior art processes (b) and (c), which are often applied in the case of inability of using the prior art process (a), are also often incapable of being applied in the case of the rapid reaction velocity of epoxy groups with an organic acid.

Still further, the prior art process (c) is often incapable of being used on an industrial basis because of the large amount of product loss and also of the considerable load in water treatments.

As mentioned above, prior art processes (a), (b) and (c) include difficult disadvantages, respectively, in application on an industrial basis.

That is, prior art processes (a), (b) and (c) have been industrially incapable of being used in the cases where an epoxy compound has properties such that a crude reaction solution can not be refined by distillation alone because of polymerization, side reaction and the epoxy group tends to rapidly react with organic acid and/or water.

Processes for the preparation of a purified 3,4-epoxycyclohexyl methyl(meth)acrylate are described in the parent application EP 92 106 244.4.

The object of the present invention is to provide a composition comprising 3,4-epoxycyclohexenyl methyl(meth)acrylate which is stable against decolouration.

### SUMMARY OF THE INVENTION

The present invention provides a composition comprising 3,4-epoxycyclohexyl methyl (meth)acrylate composition and an organic phosphorous compound represented by general formulae (I) and/or (II) described hereinafter;

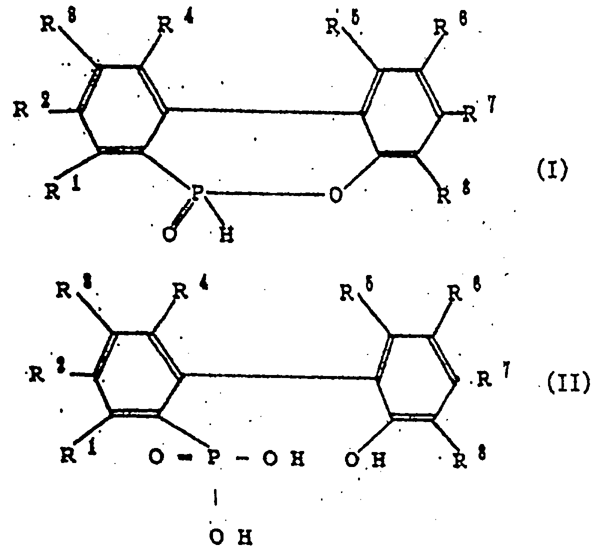

The 3, 4-epoxycyclohexyl methyl (meth)acrylate has a heptane test value of not more than 0.1 %.

### DETAILED DESCRIPTION OF THE INVENTION

The present invention is described hereinafter in more detail.

There is provided a 3,4-epoxycyclohexyl methyl (meth)acrylate composition including from 0.01 to 0.1 parts by weight of an organic phosphorous compound represented by general formulae (I) and/or (II) described hereinafter : 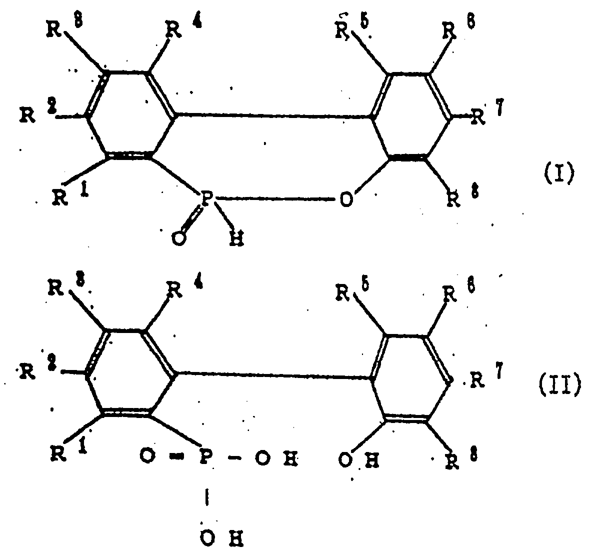 wherein R¹ to R⁸ are each independently hydrogen, a halogen, and/or a monovalent aliphatic or aromatic substituent group having from 1 to 10 carbons.

The organic phosphorous compounds are used for the purpose of preventing coloring of 3,4-epoxycyclohexyl methyl (meth)acrylate.

As described previously, it is known that 3,4-epoxycyclohexyl methyl (meth)acrylate is exceedingly readily colored in the preparation processes, while being stored and/or shipped because of oxidation by air or other causes. For example, it appears as a possible mechanism of coloring that 3,4-epoxycyclohexyl methyl (meth)acrylate itself polymerizes or by-reacts, even though in very minor amounts, because of its high reactivity.

The preferred color hue value in a commercially available 3,4-epoxycyclohexyl methyl (meth)acrylate is usually less than about 200 in APHA value, if taking it into consideration of the various uses, desirably less than about 100.

It may be generally considered that purification of 3,4-epoxycyclohexyl methyl (meth)acrylate by distillation is a suitable method for preventing coloring.

However, it is not preferable due to history of heating even though under reduced pressures.

Accordingly, it appears that a chemical treatment or an absorption treatment instead of distillation is more preferable.

In the chemical treatment, for example, there have been added molecular state oxygen, an oxidant such as hydrogen peroxide, a reducing agent such as sodium borohydride, sodium hydrogenated bis(2-methoxyethoxy)aluminum, a polymerization inhibitor such as hydroquinone, an antioxidant such as BHT (2,6-ditertbutyl-4-methyl-phenol), BHA (butyl hydroxy anisole), a blocking agent for metal such as EDTA, trioctyl phthalate, etc. in the preparation processes.

In the absorption treatment, for example, it is known that there have been used activated carbon which is a conventional absorbent, an activated clay, zeolite, a highly porous polymer, etc.

However, there are only very minor effects for preventing coloring or for discoloring according to such conventional chemical treatment or absorption treatment.

Furthermore, Japanese Patent Application No. 191267/1990 discloses the use of a hydrotalcite compound for removing coloring components in 3,4-epoxycyclohexyl methyl (meth)acrylate.

However, their coloring occurs again after a long time of period in the use of the compound.

The essential organic phosphorous compounds include 3,4,5,6-dibenzo-1,2-oxaphosphane-2-oxide, 9,10-dihydro-9-oxa-10-phosphaphenanthrene-10-oxide (for example, HCA which is a trade name, manufactured by Sanko Chemical, Ltd.), 6,8-dichloro-9,10-dihydro-9-oxa-10-phosphaphenanthrene-10-oxide, 6, 8-di(tert-butyl)-9,10-dihydro-9-oxa-10-phosphaphenanthrene-10-oxide, etc., and further a hydrated compound thereof.

The organic phosphorous compounds are represented by formula (I); 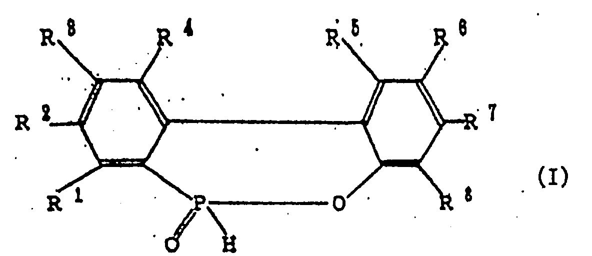 , and the hydrated compound are represented by formula (II); 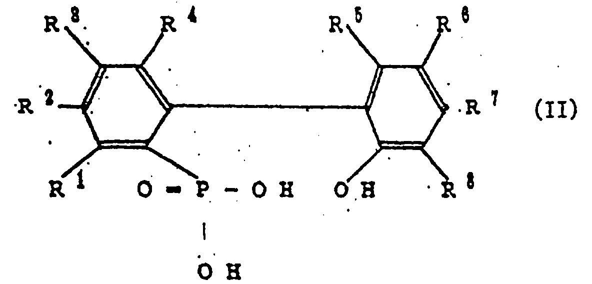 wherein R¹ to R⁸ are each independently hydrogen, halogen, and/or a monovalent aliphatic or aromatic substituent group having brom 1 to 10 carbons.

The organic phosphorous compounds can be added either in the form of powder or a solution with a solvent.

Although the organic phosphorous compounds may also be added even in any step of the preparation process, it is preferably mixed in the final product.

The amounts of the compound represented by formulae (I) and (II) are essentially from 0.01 to 0.1 parts by weight.

If the amounts used are less than 0.01 parts by weight, the effect as a polymerization inhibitor is small.

On the other hand, if the amounts used are more than 0.1 part by weight, an adverse affection is caused instead of the effect as a polymerization inhibitor.

There is also provided 3,4-epoxycyclohexyl methyl (meth)acrylate having a heptane test value of less than 0.1 % by weight.

The 3,4-epoxycyclohexyl methyl (meth)acrylate having a heptane test value of less than 0.1 % by weight can be prepared by the combined processes of the parent application EP 92 106 244.4.

The heptane test value relating to a conventional 3,4-epoxycyclohexyl methyl (meth)acrylate has been more than 0.1 % by weight, more specifically approximately 0.14 % by weight or so.

The heptane test value corresponds to the amounts of polymers having a low molecular weight composed of 3,4-epoxycyclohexyl methyl (meth)acrylate itself.

The contents of the polymers having a low molecular weight can be shown by weight % with a measuring method using n-heptane or n-hexane, in which 10 g of a product is dissolved in 100 cc of n-heptane or n-hexane and resulting suspensions are filtered and weighed.

3,4-epoxycyclohexyl methyl (meth)acrylate prepared by the combined processes of the parent application EP 92 106 244.4. exhibits usually the heptane test value of 0.02 % by weight or so.

The following Synthesis Examples, Examples and Comparative Examples are provided in order to more specifically illustrate the present invention.

### [Synthesis Example 1--- Preparation of a crude reaction solution including 3,4-epoxycyclohexyl methyl methacrylate]

A SUS 316-made reaction vessel having a capacity of 15 liter equipped with a stirrer and a jacket for cooling was charged with 3913 parts of 3-cyclohexenyl methyl (meth)acrylate (hereinafter, referred to as CHMA), 7826 parts of ethyl acetate, 5.85 parts of hydroquinone monomethylether which is a polymerization inhibitor and 2.35 parts of sodium tripolyphosphate which is a stabilizer for peracid, followed by raising the internal temperature to 45 °C.

Successively, 6329 parts of ethyl acetate solution including 30 % of peracetic acid was added dropwise over 4 hours, followed by aging for 2 hours. The internal temperature was maintained at 50 °C while adding dropwise and aging, to obtain 18076 parts of a crude reaction solution including 3,4-epoxycyclohexyl methyl methacrylate (hereinafter, referred to as METHB).

### [Example 1 --- a water extraction test with a centrifugal extractor]

The crude reaction solution obtained in synthesis Example 1 was introduced from an inlet for a light solution phase at 2108 g/minute and water was introduced from an inlet for a heavy solution phase at 3590 g/minute into a counter-current type centrifugal extractor equipped with a rotor having an outer diameter of 46 cm and an inner diameter of 25 mm which rotates at 4000 r.p.m.

And, a light solution layer was obtained at 1664 g/minute from an outlet for the light solution layer and a heavy solution layer was obtained at 4034 g/minute from an outlet for the heavy solution layer.

The light solution layer was introduced again from the inlet for the light solution layer at 2108 g/minute and water was introduced from the inlet for the heavy solution layer at 3590 g/minute.

And, a light solution layer was obtained at 1877 g/minute from the outlet for the light solution layer and a heavy solution layer was obtained at 3821 g/minute from the outlet for the heavy solution layer.

The concentrations of acetic acid and peracetic acid in the crude reaction solution before extracting with water were 10.66 % and 1.15 %, respectively.

The concentrations of acetic acid and peracetic acid in the light solution layer after extracting twice with water were 400 ppm and 150 ppm, respectively.

99 % of METHB dissolved in the crude reaction solution was recovered in the light solution layer after extracting twice with water.

### [Comparative Example 1 --- a water extraction test supposing a column type extractor]

A flask having a capacity of 2 liter equipped with a stirrer was charged with 1000 parts of a crude reaction solution obtained by the same procedures as described in Synthesis Example 1 and 1000 parts of water, followed by agitating for 60 minutes at 20 °C and settling for 30 minutes.

As the result, approximately 5 % of METHB dissolved in the crude reaction solution disappeared under the influence of the reaction of METHB with an aqueous acetic acid solution.

### [Synthesis Example 2 --- Preparation of a crude reaction solution including 3,4-epoxycyclohexyl methyl acrylate]

A SUS 316-made reaction vessel having a capacity of 20 liter equipped with a stirrer and a jacket for cooling was charged with 3000 parts of 3-cyclohexenyl methyl acrylate (hereinafter, referred to as CHAA), 11100 parts of ethyl acetate, 0.9 part of hydroquinone monomethylether which is a polymerization inhibitor and 9.0 parts of sodium tripolyphosphate, followed by introducing a mixed gas composed of oxygen and nitrogen (oxygen/nitrogen = 10/90 by volume) at 32 normal liter/hour from a tube and raising the internal temperature to 40 °C.

Successively, 5623 parts of ethyl acetate solution including 30 % of peracetic acid was added dropwise over 4 hours with a pump maintaining the reaction temperature at 40 °C, followed by aging for 5 hours after the addition of peracetic acid to complete the epoxidation reaction and to obtain 19723 parts of a crude reaction solution including 3,4-epoxycyclohexyl methyl acrylate (hereinafter, referred to as AETHB).

### [Example 2 --- a water extraction test with a centrifugal extractor]

The crude reaction solution obtained in synthesis Example 2 was introduced from an inlet for a light solution phase at 2108 g/minute and water was introduced from an inlet for a heavy solution phase at 3621 g/minute into a counter-current type centrifugal extractor equipped with a rotor having an outer diameter of 46 cm and an inner diameter of 25 mm which rotates at 4000 r.p.m.

And, a light solution layer was obtained at 1713 g/minute from an outlet for the light solution phase and a heavy solution layer was obtained at 4016 g/minute from an outlet for the heavy solution phase.

The light solution layer was introduced again from the inlet for the light solution phase at 2108 g/minute and water was introduced from the inlet for the heavy solution phase at 3590 g/minute.

And, a light solution layer was obtained at 1865 g/minute from the outlet for the light solution phase and a heavy solution layer was obtained at 3833 g/minute from the outlet for the heavy solution phase.

The concentrations of acetic acid and peracetic acid in the crude reaction solution before extracting with water were 8.96 % and 1.51 %, respectively.

The concentrations of acetic acid and peracetic acid in the light solution layer after extracting twice with water were 400 ppm and 150 ppm, respectively.

99 % of AETHB dissolved in the crude reaction solution was recovered in the light solution layer after extracting in twice with water.

### [Example 3 --- an alkali neutralization test]

A SUS 316-made vessel for mixing equipped with a stirrer and a jacket for cooling having a capacity of 15 liter was charged with 3000 parts of the light solution layer obtained in Example 2, successively, 3000 parts of an aqueous solution including 1 % of NaOH was charged into the vessel, followed by agitating for 1 hour maintaining at the temperature of 10 °C.

After settling for 30 minutes, 2790 parts of an upper solution layer was separated from a lower solution layer.

The concentrations of acetic acid and peracetic acid in the upper solution layer were less than 100 ppm, respectively.

### [Example 4 --- an alkali neutralization test]

The same procedures as described in Example 3 were repeated, except that there was charged 3000 parts of an aqueous solution including 0.5 % of NaOH.

The concentrations of acetic acid and peracetic acid in the upper solution layer were less than 100 ppm, respectively.

### [Example 5 --- an alkali neutralization test]

The same procedures as described in Example 5 were repeated, except that there was charged 3000 parts of an aqueous solution including 0.1 % of NaOH.

The concentrations of acetic acid and peracetic acid in the upper solution layer were less than 100 ppm, respectively.

### [Example 6 --- an alkali neutralization test]

The same procedures as described in Example 3 were repeated, except that there was charged 300 parts of an aqueous solution including 1 % of NaOH. The concentrations of acetic acid and peracetic acid in the upper solution layer were less than 100 ppm, respectively.

### [Comparative Example 2 --- a water extraction test supposing a column type extractor]

A SUS316-made mixing vessel having a capacity of 15 liter equipped with a stirrer was charged with 3000 parts of a crude reaction solution obtained by the same procedures as described in Synthesis Example 2 and 3000 parts of a distilled water, followed by agitating for 60 minutes at 10 °C and settling for 60 minutes, resulting in two solution layers.

2790 parts of the light solution layer was separated from the heavy solution layer.

As the result, approximately 5 % of AETHB dissolved in the crude reaction solution disappeared under the influence of the reaction of AETHB with an aqueous acetic acid.

### [Comparative Example 3 --- an evaporation test after a water extraction test supposing a column type extractor and without neutralization with an aqueous alkali solution]

0.21 part of hydroquinone monomethylether was added in 2790 parts of the upper solution layer obtained in Comparative Example 2, the solution was supplied into a SUS-made Smith falling film type evaporator maintained at the temperature of 60 °C and the reduced pressure of 150 mm Hg supplying a mixed gas composed of oxygen and nitrogen at 32 liter/hour from the bottom of the evaporator, to which a line for discharging a liquid is connected, to obtain a solution including 5 % of low boiling components.

Successively, the solution discharged from the bottom was supplied again into the SUS-made Smith falling film type evaporator at the temperature of 60 °C and the reduced pressure of 40 mm Hg supplying a mixed gas composed of oxygen and nitrogen at 32 liter/hour from the bottom of the evaporator to which a line for discharging a liquid is connected, to obtain 538 parts of a product including not more than 1 % of low boiling ingredients.

The concentration of AETHB in the product was 86.5 % by a gas chromatography analysis.

### [Example 7 --- an evaporation test]

0.21 part of hydroquinone monomethylether was added in 2790 parts of the upper solution layer obtained in Example 3, the solution was supplied into a SUS-made Smith falling film type evaporator maintained at the temperature of 60 °C and the reduced pressure of 150 mm Hg supplying a mixed gas composed of oxygen and nitrogen at 32 liter/hour from the bottom of the evaporator, to which a line for discharging a liquid is connected, to obtain a solution including 5 % of low-boiling ingredients.

Successively, the solution discharged from the bottom was supplied again into the SUS-made Smith falling film type evaporator at the temperature of 60 °C and the reduced pressure of 40 mm Hg supplying a mixed gas composed of oxygen and nitrogen at 32 liter/hour from the bottom of the evaporator to which a line for discharging a liquid is connected, to obtain 538 parts of a product including not more than 1 % of low-boiling ingredients.

The concentration of AETHB in the product was 96.4 % by a gas chromatography analysis.

The heptane test value of the product was 0.02 %.

### [Example 8 --- an evaporation test]

The same procedures as described in Example 7 were repeated, except that the upper solution layer obtained in Example 4 was used to obtain 538 parts of a product. The concentration of AETHB in the product was 95.7 % by a gas chromatography analysis.

The heptane test value of the product was 0.02 %.

### [Comparative Example 4 --- an evaporation test without neutralization with alkali after extracting with water]

0.21 part of hydroquinone monomethylether was added in 2300 parts of the same upper solution layer as obtained in Example 1, the solution was supplied into a SUS-made Smith falling film type evaporator maintained at the temperature of 60 °C and the reduced pressure of 150 mm Hg supplying a mixed gas composed of oxygen and nitrogen at 32 liter/hour from the bottom of the evaporator, to which a line for discharging a liquid is connected, to obtain a solution including 5 % of low-boiling ingredients.

Successively, the solution discharged from the bottom was supplied again into the SUS-made Smith falling film type evaporator at the temperature of 60 °C and the reduced pressure of 40 mm Hg supplying a mixed gas composed of oxygen and nitrogen at 32 liter/hour from the bottom of the evaporator to which a line for discharging a liquid is connected, to obtain a product including not more than 1 % of low-boiling ingredients.

The concentration of METHB in the product was 89.5 % by a gas chromatography analysis.

### [Example 9 --- a coloring test after mixing an organic phosphorous compound]

0.1 part of 9,10-dihydro-9-oxa-10-phosphaphenanthrene-10-oxide was mixed with 100 parts of the product which has a color hue of 40 in APHA value obtained in Example 7.

The mixture was stored for three months at 30 °C. As the result, the APHA value changed to 50.

### [Comparative Example 4 --- a coloring test without mixing an organic phosphorous compound]

The product which has a color hue of 40 in APHA value obtained in Example 7 without mixing any additives was stored for three months at 30 °C. As the result, the APHA value changed to 400.

### [Comparative Example 5 --- a coloring test after mixing large amounts of an organic phosphorous compound]

1 part of 9,10-dihydro-9-oxa-10-phosphaphenanthrene-10-oxide was mixed with 100 parts of the product which has AETHB contents of 96.4 obtained in Example 7. The mixture was stored for three months at 30 °C. As the result, the AETHB contents changed to 91.2 %.

### [Comparative Example 6 --- a coloring test after mixing small amounts of an organic phosphorous compound]

0.001 part of 9,10-dihydro-9-oxa-10-phosphaphenanthrene-10-oxide was mixed with 100 parts of the product which has a color hue of 40 in APHA value obtained in Example 7. The mixture was stored for three months at 30 °C. As the result, the APHA value changed to 130.

### [Comparative Example 7 --- a coloring test after mixing of a conventional coloring inhibitor]

0.1 part of BHT which is an antioxidant was mixed with 100 parts of the product which has a color hue of 40 in APHA value obtained in Example 7. The mixture was stored for three months at 30 °C. As the result, the APHA value changed to 150.

### [Comparative Example 8 --- a coloring test after mixing of a conventional coloring inhibitor]

0.1 part of BHA which is an antioxidant was mixed with 100 parts of the product which has a color hue of 40 in APHA value obtained in Example 7. The mixture was stored for three months at 30 °C. As the result, the APHA value changed to 180.

### [Comparative Example 9 --- a coloring test after mixing of a conventional coloring inhibitor]

0.1 part of Irganox (manufactured by Ciba-Geigy Corp.) which is an antioxidant was mixed with 100 parts of the product which has a color hue of 40 in APHA value obtained in Example 7.

The mixture was stored for three months at 30 °C. As the result, the APHA value changed to 160.

### [Comparative Example 10 --- a coloring test after mixing of a conventional coloring inhibitor]

0.1 part of Sanol (manufactured by Ciba-Geigy Corp.) which is an antioxidant was mixed with 100 parts of the product which has a color hue of 40 in APHA value obtained in Example 7.

The mixture was stored for three months at 30 °C. As the result, the APHA value changed to 350.

### [Comparative Example 11 --- an evaporation test after alkali neutraliza tion treatments thrice without extraction with water]

3000 parts of a crude reaction solution obtained by the same procedures as described in Synthesis Example 2 was mixed with 2500 parts of an aqueous solution including 10 % of NaOH, followed by stirring for 30 minutes and settling for 30 minutes, and then followed by separating the reaction solution.

The separated solution was mixed again with 2500 parts of an aqueous solution including 10 % of NaOH, followed by stirring for 30 minutes and settling for 30 minutes, and then followed by separating the reaction solution.

The contents of peracetic acid in the separated solution were 0.02 %, and acetic acid was completely removed.

The separated solution was further mixed with 2500 parts of an aqueous solution including 1 % of NaOH, followed by stirring for 30 minutes and settling for 30 minutes, and then followed by separating the reaction solution. The contents of peracetic acid in the separated solution were less than 100 ppm.

The same procedures as described in Example 7 were repeated, except that the solution obtained in the alkali neutralization treatments was used to obtain a product.

The concentration of AETHB in the product was 94.5 % by a gas chromatography analysis. And, the contents of ethyl acetate, CHAA and other components were 1.8 %, 1.0 % and 2.7 %, respectively.

The heptane test value of the product was 0.25 %, despite of evaporation in peracetic acid contents of less than 100 ppm and complete removal of acetic acid, because of a long contacting time under the coexistence of peracetic acid, acetic acid and water.

While the invention has been described in detail and with reference to specific embodiments thereof, it will be apparent to one skilled in the art that various changes and modifications can be made therein without departing from the spirit and scope thereof.

## Claims (Claims for the following Contracting State(s): DE, FR, GB, NL)

1. A composition comprising 3,4-epoxycyclohexyl methyl (meth)acrylate and from 0.01 to 0.1 part by weight of an organic phosporous compound represented by the formulae (I) and/or (II): 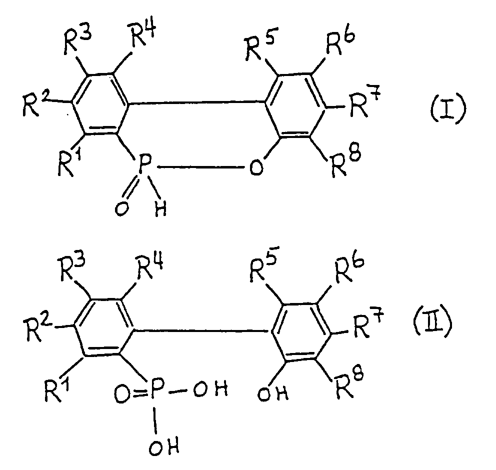 wherein, R¹ to R⁸ each independently are hydrogen, halogen, and/or a monovalent aliphatic or aromatic substituent group having from 1 to 10 carbons.

## Claims (Claims for the following Contracting State(s): ES)

1. A process for the preparation of a composition comprising 3,4-epoxycyclohexyl methyl (meth)acrylate and from 0.01 to 0.1 part by weight of an organic phosphorous compound represented by the formula (I) and/or (II): 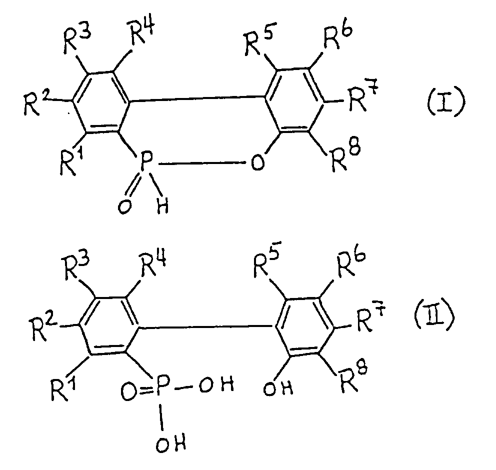 wherein R¹ to R⁸ each independently are hydrogen, halogen, and/or a monovalent aliphatic or aromatic substituent group having from 1 to 10 carbons wherein the above organic phosphorous compounds(s) is (are) added in the form of a powder or a solution with a solvent and mixed with the 3,4-epoxycyclohexyl methyl (meth)acrylate.

## Patentansprüche (Patentansprüche für folgende(n) Vertragsstaat(en): DE, FR, GB, NL)

1. Zusammensetzung, umfassend 3,4-Epoxycyclohexylmethyl(meth)acrylat und 0,01 bis 0,1 Gew.-Teile einer organischen Phosphorverbindung, dargestellt durch die Formeln (I) und/oder (II): 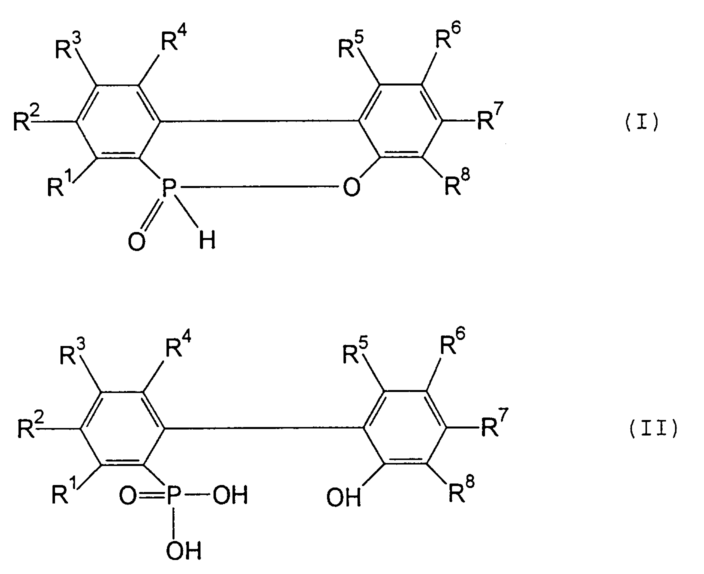 worin R¹ bis R⁸ unabhängig jeweils Wasserstoff, Halogen und/oder eine einwertige aliphatische oder aromatische Substituentengruppe mit 1 bis 10 Kohlenstoffatomen sind.

## Patentansprüche (Patentansprüche für folgende(n) Vertragsstaat(en): ES)

1. Verfahren zur Herstellung einer Zusammensetzung, umfassend 3,4-Epoxycyclohexylmethyl(meth)acrylat und 0,01 bis 0,1 Gew.-Teile einer organischen Phosphorverbindung, dargestellt durch die Formeln (I) und/oder (II): 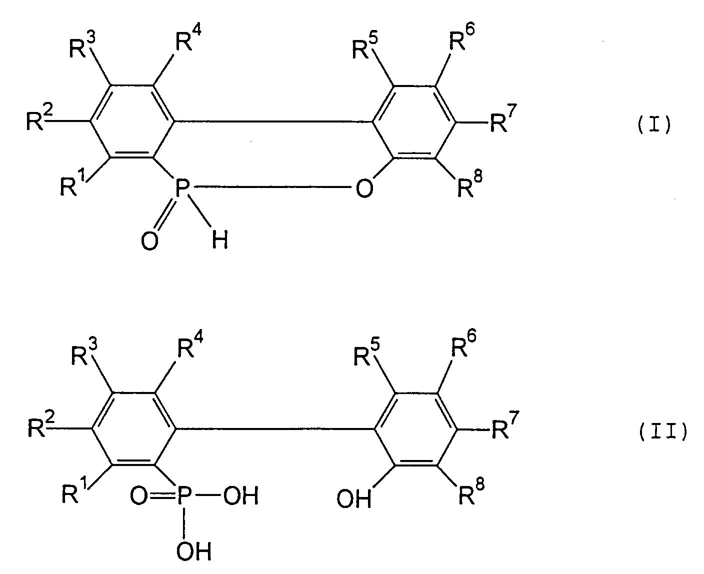 worin R¹ bis R⁸ unabhängig jeweils Wasserstoff, Halogen und/oder eine einwertige aliphatische oder aromatische Substituentengruppe mit 1 bis 10 Kohlenstoffatomen sind, worin der(die) obige(n) organische(n) Phosphorverbindung(en) in Form eines Pulvers oder einer Lösung zu einem Lösungsmittel hinzugegeben und mit dem 3,4-Epoxycyclohexylmethyl(meth)acrylat vermischt wird(werden).

## Revendications (Revendications pour l'(les) Etat(s) contractant(s) suivant(s): DE, FR, GB, NL)

1. Composition comprenant du (méth)acrylate de 3,4-époxycyclohexylméthyle et de 0,01 à 0,1 parties en poids d'un composé organique du phosphore représenté par les formules (I) et/ou (II) : 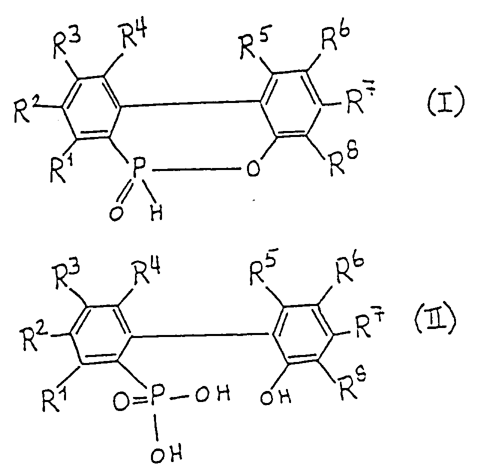 dans lesquelles R¹ à R⁸ sont chacun, de manière indépendante, un hydrogène, un halogène et/ou un groupe substituant monovalent aliphatique ou aromatique ayant de 1 à 10 atomes de carbone.

## Revendications (Revendications pour l'(les) Etat(s) contractant(s) suivant(s): ES)

1. Procédé de préparation d'une composition comprenant du (méth)acrylate de 3,4-époxycyclohexylméthyle et de 0,01 à 0,1 parties en poids d'un composé organique du phosphore représenté par la formule (I) et/ou (II) : 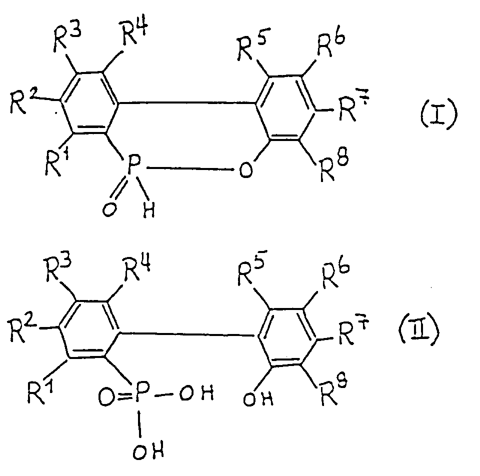 dans laquelle R¹ à R⁸ sont chacun, de manière indépendante, un hydrogène, un halogène et/ou un groupe substituant monovalent aliphatique ou aromatique ayant de 1 à 10 atomes de carbone, dans lequel le ou les composés organiques du phosphore ci-dessus est (sont) ajouté(s) sous la forme d'une poudre ou d'une solution avec un solvant et mélangés avec le (méth)acrylate de 3,4-époxycyclo- hexylméthyle.
